# EUROPEAN PATENT APPLICATION

(11) **EP 4 461 132 A1**
(43) Date of publication of application: **13.11.2024**
(21) Application number: 23918900.4
(22) Date of filing: 26.10.2023
(51) Int. Cl.: A01K 1/01

(54) **DEODORIZATION APPARATUS AND CAT TOILET**

(30) Priority: 07.03.2023 CN 202310208614
(71) Applicant: PETKIT NETWORK TECHNOLOGY (SHANGHAI) CO., LTD., Shanghai 201100 (CN)
(72) Inventor: SHI, Xin, Shanghai 201100 (CN); GUO, Weike, Shanghai 201100 (CN); JIANG, Lihua, Shanghai 201100 (CN); MA, Yunxin, Shanghai 201100 (CN); MA, Xiaoqing, Shanghai 201100 (CN); ZHANG, Junchao, Shanghai 201100 (CN)
(74) Representative: Cabinet Beaumont
(86) International application number: PCT/CN2023/126878
(87) International publication number: WO 2024/183295

(57) **Abstract**

Provided are a deodorization device and a cat toilet. The deodorization device is installed in an installation hole of a drum and includes a deodorization component rotatably disposed in the installation hole. The deodorization component includes a deodorization liquid bottle, a long cotton core inserted into the deodorization liquid bottle, a short cotton core overlapping the long cotton core, and a spray assembly. The spray assembly is connected to the short cotton core. The short cotton core is configured to deliver deodorization liquid in the deodorization liquid bottle to the spray assembly through the long cotton core. The spray assembly is configured to spray the deodorization liquid into the drum.

## Description

This application claims priority to Chinese Patent Application No. 202310208614.8 filed on Mar. 07, 2023, the disclosure of which is incorporated herein by reference in its entirety.

### TECHNICAL FIELD

The present application relates to the technical field of pet supplies, for example, a deodorization device and a cat toilet.

### BACKGROUND

To meet the requirements of families keeping pets, the pet industry provides corresponding pet supplies according to different biological characteristics of pets. For example, the biological instinct of a pet cat has a relatively high requirement for a defecation environment, and the pet cat requires the excreta to be buried in sand grains to avoid being smelled by a natural enemy. Thus, a keeper of the pet cat provides the pet cat with a litter basin, also known as a cat toilet, and places litter in the cat toilet. Having finished excreting in the litter, the pet cat paws the litter to cover up the excreta. However, the smell produced by the excreta lingers, affecting the freshness of the air and producing harmful bacteria. For cat toilets in the related art, especially cat toilets provided with a drum, how to stably and all-roundly deodorize the interiors of the cat toilets and how to still deodorize in real time in a rotation process of the drum are urgent problems to be solved.

### SUMMARY

The present application provides a deodorization device and a cat toilet so that stable and all-round deodorization can be achieved, and the freshness of the air can be improved.

An embodiment of the present application provides a deodorization device installed in an installation hole of a drum. The deodorization device includes a deodorization component rotatably disposed in the installation hole. The deodorization component includes a deodorization liquid bottle, a long cotton core inserted into the deodorization liquid bottle, a short cotton core overlapping the long cotton core, and a spray assembly. The spray assembly is connected to the short cotton core. The short cotton core is configured to deliver deodorization liquid in the deodorization liquid bottle to the spray assembly through the long cotton core. The spray assembly is configured to spray the deodorization liquid into the drum.

An embodiment of the present application further provides a cat toilet including a drum and the preceding deodorization device. The deodorization device is installed in an installation hole of the drum.

### BRIEF DESCRIPTION OF DRAWINGS

FIG. 1 is a view illustrating the structure of a deodorization device and a drum according to an embodiment of the present application.
FIG. 2 is an exploded view of a deodorization device and a drum according to an embodiment of the present application.
FIG. 3 is a side view of a deodorization device according to an embodiment of the present application.
FIG. 4 is a first axonometric view of a deodorization component according to an embodiment of the present application.
FIG. 5 is a second axonometric view of a deodorization component according to an embodiment of the present application.
FIG. 6 is a sectional view of a deodorization device according to an embodiment of the present application.
FIG. 7 is an exploded view of a deodorization component according to an embodiment of the present application.
FIG. 8 is an exploded view of a rotation chassis according to an embodiment of the present application.

### Reference list

- 100: drum
- 101: installation hole
- 10: deodorization component
- 20: rotation chassis
- 111: rear cover
- 112: housing
- 1121: spray outlet
- 113: inner frame
- 12: hanger
- 13: printed circuit board (PCB)
- 14: main board
- 15: lighting lamp
- 16: battery
- 171: deodorization liquid bottle
- 172: long cotton core
- 173: short cotton core
- 181: spray head
- 182: compression spring
- 183: atomization sheet
- 184: atomization cover plate
- 21: rotation inner ring
- 211: installation portion
- 22: counterweight block
- 23: bearing platen
- 24: bearing
- 25: rotation outer ring
- 26: rotation hanger
- 261: insertion groove

### DETAILED DESCRIPTION

In the description of the present application, terms "joined", "connected" and "fixed" are to be understood in a broad sense unless otherwise expressly specified and limited. For example, the term "connected" may refer to "fixedly connected" or "detachably connected", may refer to "mechanically connected" or "electrically connected", or may refer to "connected directly", "connected indirectly through an intermediary", or "connected inside two elements" or "an interaction relation between two elements". For those of ordinary skill in the art, specific meanings of the preceding terms in the present application may be understood based on specific situations.

In the description of the present application, unless otherwise expressly specified and limited, when a first feature is described as "on" or "below" a second feature, the first feature and the second feature may be in direct contact or be in contact via another feature between the two features instead of being in direct contact. Moreover, when the first feature is described as "on", "above", or "over" the second feature, the first feature is right on, above, or over the second feature, the first feature is obliquely on, above, or over the second feature, or the first feature is simply at a higher level than the second feature. When the first feature is described as "under", "below", or "underneath" the second feature, the first feature is right under, below, or underneath the second feature, the first feature is obliquely under, below, or underneath the second feature, or the first feature is simply at a lower level than the second feature.

According to the biological instinct, a pet cat has a relatively high requirement for a defecation environment and requires the excreta to be buried in sand grains to avoid being smelled by a natural enemy. Thus, a keeper of the pet cat provides the pet cat with a litter basin, also known as a cat toilet, and places litter in the cat toilet. Having finished excreting in the litter, the pet cat paws the litter to cover up the excreta. However, the smell produced by the excreta lingers, affecting the freshness of the air and producing harmful bacteria. For cat toilets in the related art, especially cat toilets provided with a drum, how to stably and all-roundly deodorize the interiors of the cat toilets and how to still deodorize in real time in a rotation process of the drum are urgent problems to be solved.

The technical solutions in the embodiments of the present application are further described below through specific implementations in conjunction with the drawings.

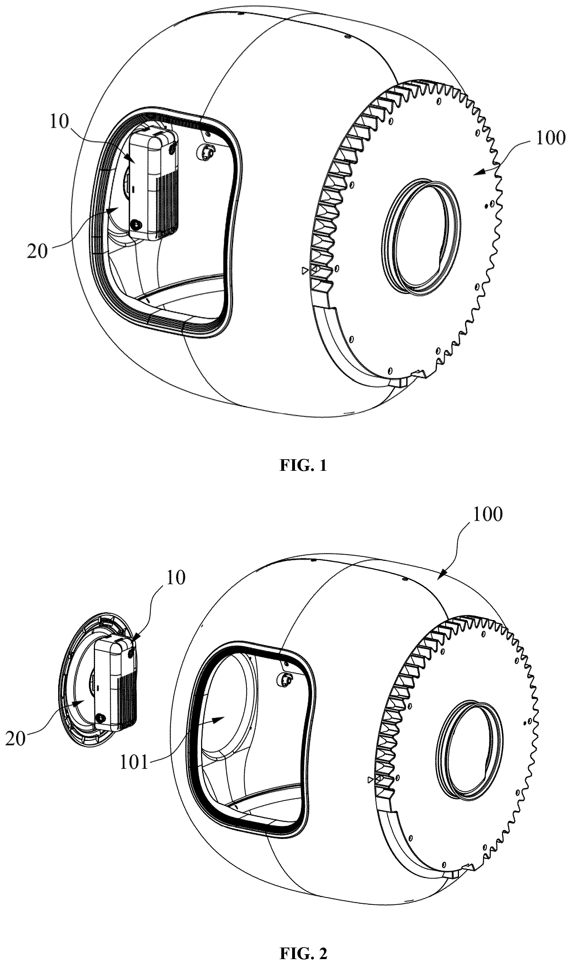

As shown in FIGS. 1 to 8, this embodiment provides a deodorization device and a cat toilet. In this embodiment, the deodorization device is installed in an installation hole 101 of a drum 100 and includes a deodorization component 10 rotatably disposed in the installation hole 101. The deodorization component 10 includes a deodorization liquid bottle 171, a long cotton core 172 inserted into the deodorization liquid bottle 171, a short cotton core 173 overlapping the long cotton core 172, and a spray assembly. The spray assembly is connected to the short cotton core 173. The short cotton core 173 delivers deodorization liquid in the deodorization liquid bottle 171 to the spray assembly through the long cotton core 172. The spray assembly can spray the deodorization liquid into the drum 100. This embodiment further provides a cat toilet. The cat toilet includes the deodorization device.

In this embodiment, the interior of the drum 100 is deodorized in real time through the deodorization device, and the deodorization component 10 is rotatably connected to the drum 100 in the deodorization device so that when the drum 100 rotates, the position of the deodorization component 10 can remain unchanged, avoiding leaking the deodorization liquid in the deodorization liquid bottle 171 in the deodorization component 10 and ensuring smooth deodorization operation of the deodorization component 10. In this embodiment, the long cotton core 172 and the short cotton core 173 overlap with each other, and the long cotton core 172 is inserted into the deodorization liquid bottle 171 so that the deodorization liquid in the deodorization liquid bottle 171 can be delivered to the short cotton core 173 through capillary action, and the deodorization liquid can be sprayed into the drum 100 through the spray assembly under the connection action of the short cotton core 172 and the spray assembly. In this way, the interior of the drum 100 can be all-roundly deodorized, and when the drum 100 rotates, stable and all-round deodorization can still be performed, and the freshness of the air can be improved. The cat toilet provided with the deodorization device can also achieve a stable and overall deodorization effect.

The specific structure of the deodorization device in this embodiment is described below.

As shown in FIGS. 1 to 3, in this embodiment, the deodorization device includes the deodorization component 10 and a rotation chassis 20. In an embodiment, the rotation chassis 20 is installed in the installation hole 101 of the drum 100, the deodorization component 10 is connected to the rotation chassis 20, and the rotation chassis 20 is rotatably disposed in the installation hole 101. Thus, the deodorization function of the deodorization device is fulfilled through the deodorization component 10, and the deodorization component 10 rotates with respect to the drum 100 by using the rotation chassis 20.

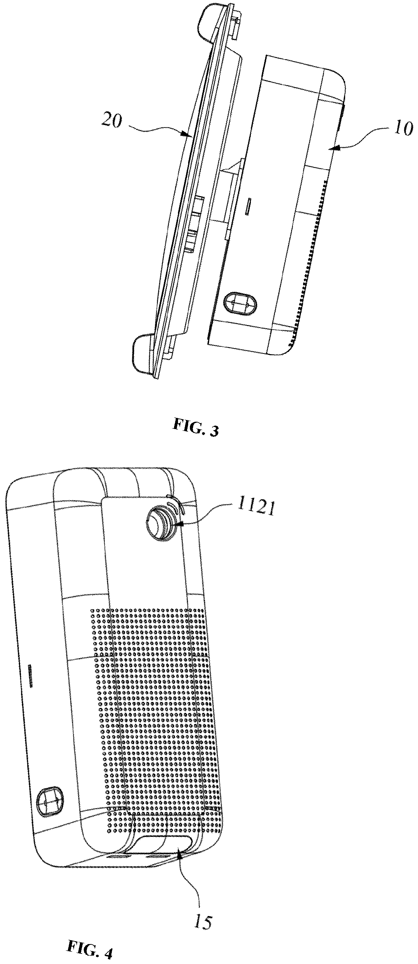

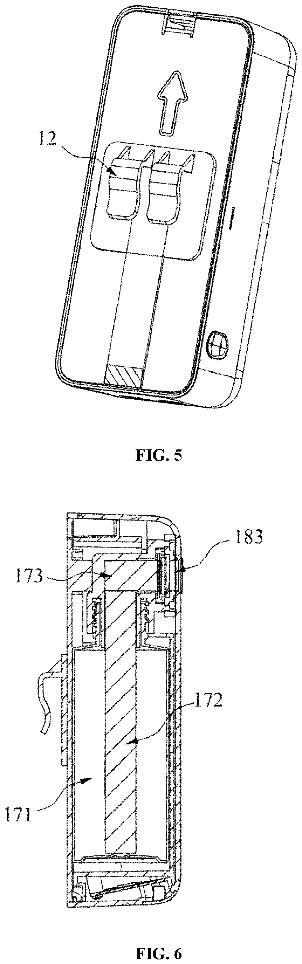

In an embodiment, as shown in FIGS. 4 to 7, the deodorization component 10 includes a rear cover 111, a housing 112, an inner frame 113, a hanger 12, a PCB board 13, a main board 14 and a lighting lamp 15, and the housing 112 is provided with a spray outlet 1121. In this embodiment, an accommodation space is formed by enclosing the inner side of the rear cover 111 and the housing 112, and the hanger 12 is disposed on the outer side of the rear cover 111 and configured to connect the rotation chassis 20. In an embodiment, the rear cover 111 is provided with an engagement block, the housing 112 is provided with an engagement opening, and the engagement block can snap into the engagement opening to make the rear back 111 and the housing 112 detachably connected, making it easier to overhaul the interior of the deodorization component 10. As shown in FIG. 5, the hanger 12 includes a square plate and two insertion blocks. The two insertion blocks are symmetrically disposed on the square plate, and each insertion block is provided with a connection portion and an abutment portion. The connection portion is connected to the square plate, and the abutment portion can be pressed against the square plate. In an embodiment, as shown in FIG. 4, the housing 112 is provided with the spray outlet 1121, and the spray outlet 112 passes through the housing 112 to facilitate the subsequent spray of the deodorization liquid.

In an embodiment, the inner frame 113 is disposed in the accommodation space. In this embodiment, the inner frame 113 is provided with an avoidance opening to allow the engagement block on the rear cover 111 to pass through the avoidance opening to be connected to the engagement opening of the housing 112. In other embodiments, the inner frame 113 may also be provided with a locking groove so that the engagement block can be inserted into the locking groove after snapping into the engagement opening, ensuring the tight connection between the rear cover 111, the inner frame 113 and the housing 112. In an embodiment, the PCB board 13 is disposed on the side surface of the inner frame 113, and the main board 14 is disposed at the bottom of the inner frame 113 to transmit an electronic signal of the deodorization component 10 stably.

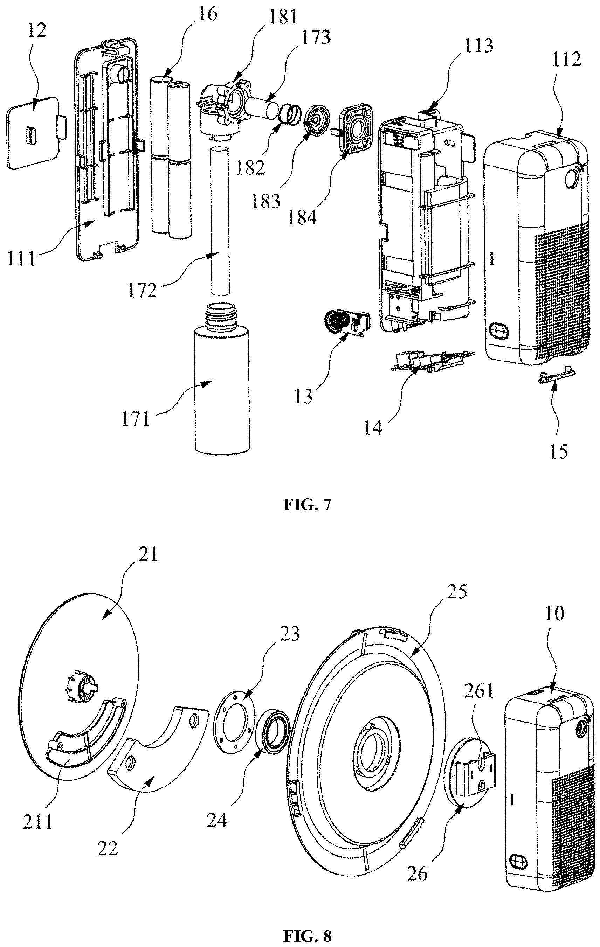

As shown in FIGS. 6 and 7, the deodorization component 10 further includes the lighting lamp 15, a battery 16, the deodorization liquid bottle 171, the long cotton core 172, the short cotton core 173 and the spray assembly. As shown in FIG. 4, the lighting lamp 15 is disposed at the bottom of the housing 112 and configured to identify the position of the deodorization component 10 in the drum 100 to locate the position of the deodorization component 10 in a dim environment within the drum 100 accurately. In other embodiments, the working state of the deodorization component 10 may also be observed through the lighting lamp 15 to help timely maintain or replace the deodorization component 10. The battery 16 is disposed in the accommodation space, is installed on the inner side of the rear cover 111, is electrically connected to the spray assembly and is configured to drive the spray assembly.

In an embodiment, the deodorization liquid bottle 171, the long cotton core 172, the short cotton core 173 and the spray assembly are disposed on the inner frame 113 in the accommodation space. The specific shape of the inner frame 113 is adapted to the deodorization liquid bottle 171, the long cotton core 172, the short cotton core 173 and the spray assembly to increase the space utilization rate of the accommodation space so that the deodorization component 10 can have a compact overall structure, facilitating storage in the drum 100. In an embodiment, a first end of the long cotton core 172 abuts against the bottom surface of the inner cavity of the deodorization liquid bottle 171, and a second end of the long cotton core 172 passes through the mouth of the deodorization liquid bottle 171 and overlaps one end of the short cotton core 173. As shown in FIG. 6, the long cotton core 172 is disposed along an axial direction of the deodorization liquid bottle 171, and the short cotton core 173 is vertical to the long cotton core 172 to help deliver the deodorization liquid in the deodorization liquid bottle 171 to the long cotton core 172 through the capillary action and then to the short cotton core 173. The lengths of the long cotton core 172 and the short cotton core 173 may be set according to requirements. This is not repeated herein.

In an embodiment, the spray assembly is connected to the short cotton core 173 so that the deodorization liquid can be sprayed into the drum 100 through the spray outlet 1121. The spray assembly includes a spray head 181, a compression spring 182, an atomization sheet 183 and an atomization cover plate 184. As shown in FIG. 7, the spray head 181 is connected to the short cotton core 173. In this embodiment, the spray head 181 is sleeved on the outer side of the short cotton core 173 and the outer side of the mouth of the deodorization liquid bottle 171 to enable the spray head 181 to be sleeved on the outer side of a joint between the long cotton core 172 and the short cotton core 173 so as to ensure stable delivery of the deodorization liquid to the spray assembly and achieve tight connection between the spray assembly and the deodorization liquid bottle 171. In an embodiment, the compression spring 182 is sleeved on the outer side of the short cotton core 173, the atomization sheet 183 seals and covers one end of the short cotton core 173, and the atomization sheet 183 is secured to the spray head 181 by the atomization cover plate 184. In this embodiment, the spray assembly can spray the deodorization liquid delivered by the short cotton core 173 into the drum 100, and the deodorization liquid is crushed into liquid particles by the atomization sheet 183 and then is further sprayed into the drum 100 to achieve all-round deodorization. In other embodiments, other spray devices may also be selected for all-roundly deodorizing the interior of the drum 100 by using the deodorization liquid. This is not repeated herein.

As shown in FIG. 8, the rotation chassis 20 includes a rotation inner ring 21, a counterweight block 22, a bearing platen 23, a bearing 24, a rotation outer ring 25 and a rotation hanger 26, the rotation inner ring 21 is provided with an installation portion 211, and the rotation hanger 26 is provided with an insertion groove 261. In an embodiment, the rotation outer ring 25 is installed in the installation hole 101 of the drum 100, the rotation inner ring 21 is rotatably connected to the rotation outer ring 25, the counterweight block 22 is disposed on the rotation inner ring 21, the deodorization component 10 is connected to the rotation inner ring 21, and when the drum 100 drives the rotation outer ring 25 to rotate synchronously, the position of the rotation inner ring 21 remains unchanged under the action of the counterweight block 22. Thus, the deodorization component 10 is rotatably connected to the drum 100, and when the drum 100 rotates, the position of the deodorization component 10 remains unchanged under the action of the connection with the rotation inner ring 21 and the gravity action of the counterweight block 22 so that the deodorization component 10 can be always placed along a vertical direction, thereby avoiding tilting or overturning of the deodorization liquid bottle 171, avoiding leaking the deodorization liquid, ensuring that the deodorization component 10 can perform an all-round and stable deodorization operation under any circumstances of the drum 100, increasing the utilization rate of the deodorization device and greatly improving the freshness of the air.

In an embodiment, the installation portion 211 on the rotation inner ring 21 is configured as an arc-shaped structure and is adapted to the shape of the counterweight block 22. In an embodiment, the installation portion 211 is provided with a protruding block, the counterweight block 22 is provided with a through hole, the protruding block is disposed in one-to-one correspondence with the through hole, and the protruding block can be inserted into the through hole to stably connect the rotation inner ring 21 to the counterweight block 22. In an embodiment, the weight of the counterweight block 22 may be designed according to requirements to ensure that the rotation inner ring 21 can always remain upright only under the gravity action of the counterweight block 22. In this embodiment, the rotation inner ring 21 is rotatably connected to the rotation outer ring 25 through the bearing 24. In an embodiment, the inner side of the bearing 24 is sleeved on the outer side of a central shaft of the rotation inner ring 21, the bearing platen 23 is sleeved on the outer side of the bearing 24, a certain clearance is present between the bearing platen 23 and the rotation inner ring 21, and the bearing platen 23 is connected to the rotation outer ring 25 through screws to ensure the stable connection between the bearing 24 and the rotation outer ring 25 and the relative rotation of the rotation inner ring 23 with respect to the rotation outer ring 25 is achieved.

In an embodiment, the central shaft of the rotation inner ring 21 is provided with an accommodation groove, two cylindrical grooves are disposed within the accommodation groove, and the inner wall of each cylindrical groove is provided with threads. In an embodiment, a connection strip extends from the opening of the accommodation groove. Correspondingly, the rotation hanger 26 includes a bottom plate and a socket sleeve. The socket sleeve is secured to one side of the bottom plate, and the bottom plate is provided with a connection hole and two thread holes, and the connection hole and the two thread holes pass through the bottom plate. The connection strip can be inserted into the connection hole, and the screws sequentially pass through the two thread holes on the bottom plate and the two cylindrical grooves on the rotation inner ring 21 so that the bottom plate can be securely connected to the rotation inner ring 21. In this embodiment, the two thread holes pass through the socket sleeve, so the screws can pass through the outer side of the socket sleeve and tightly connect the socket sleeve, the bottom plate and the rotation inner ring 21 to stably connect the rotation hanger 26 to the rotation inner ring 21. Moreover, when the rotation inner ring 21 rotates with respect to the rotation outer ring 25, the rotation hanger 26 can rotate with the rotation inner ring 21 synchronously and does not interfere with the rotation outer ring 25. In an embodiment, the insertion groove 261 is disposed on the socket sleeve, the opening of the insertion groove 261 faces upward, and the hanger 12 of the deodorization component 10 can be inserted into the insertion groove 261 from top to bottom so that the abutment portion of the each insertion block in the hanger 12 can press the sidewall of the insertion groove 261 against the square plate, stably connecting the hanger 12 to the rotation hanger 26 and further stably connecting the deodorization component 10 to the rotation inner ring 21. Moreover, under the action of the bearing 24, the deodorization component 10 can be rotatably connected to the rotation outer ring 25, making the deodorization component 10 rotate with respect to the drum 10 and achieving the all-round and stable deodorization effect.

In this embodiment, the cat toilet is further provided and includes the preceding deodorization device to all-roundly and stably deodorize the interior of the cat toilet.

## Claims

1. A deodorization device, installed in an installation hole (101) of a drum (100), and comprising:
a deodorization component (10) rotatably disposed in the installation hole (101), wherein the deodorization component (10) comprises a deodorization liquid bottle (171), a long cotton core (172) inserted into the deodorization liquid bottle (171), a short cotton core (173) overlapping the long cotton core (172), and a spray assembly, the spray assembly is connected to the short cotton core (173), the short cotton core (173) is configured to deliver deodorization liquid in the deodorization liquid bottle (171) to the spray assembly through the long cotton core (172), and the spray assembly is configured to spray the deodorization liquid into the drum (100).

2. The deodorization device according to claim 1, wherein the spray assembly comprises a spray head (181) and an atomization sheet (183), the spray head (181) is sleeved on an outer side of the short cotton core (173), and the atomization sheet (183) is connected to the spray head (181) and configured to crush the deodorization liquid into liquid particles.

3. The deodorization device according to claim 1, further comprising a rotation chassis (20), wherein the deodorization component (10) is connected to the rotation chassis (20), and the rotation chassis (20) is installed in the installation hole (101).

4. The deodorization device according to claim 3, wherein the rotation chassis (20) comprises a rotation inner ring (21), a counterweight block (22) and a rotation outer ring (25), the rotation outer ring (25) is installed in the installation hole (101), the rotation inner ring (21) is rotatably connected to the rotation outer ring (25), the counterweight block (22) is disposed on the rotation inner ring (21), the deodorization component (10) is connected to the rotation inner ring (21), and the rotation inner ring (21) is configured to remain unchanged at a position under an action of the counterweight block (22) in response to the drum (100) driving the rotation outer ring (25) to rotate synchronously.

5. The deodorization device according to claim 4, wherein the deodorization component (10) is provided with a hanger (12), the rotation chassis (20) is provided with a rotation hanger (26) connected to the rotation inner ring (21), the rotation hanger (26) is provided with an insertion groove (261), and the hanger (12) is inserted into the insertion groove (261).

6. The deodorization device according to claim 5, wherein the deodorization component (10) comprises a rear cover (111) and a housing (112), the hanger (12) is disposed on an outer side of the rear cover (111), an inner side of the rear cover (111) encloses the housing (112) to form an accommodation space, and the deodorization liquid bottle (171), the long cotton core (172), the short cotton core (173) and the spray assembly are disposed in the accommodation space.

7. The deodorization device according to claim 6, wherein the housing (112) is provided with a spray outlet (1121), and the spray assembly is configured to spray the deodorization liquid into the drum (100) through the spray outlet (1121).

8. The deodorization device according to any one of claims 1 to 7, wherein the deodorization component (10) is provided with a lighting lamp (15), and the lighting lamp (15) is configured to identify a position of the deodorization component (10) in the drum (100).

9. The deodorization device according to any one of claims 1 to 7, wherein a battery (16) is disposed in the deodorization component (10) and configured to drive the spray assembly.

10. A cat toilet, comprising a drum (100) and the deodorization device according to any one of claims 1 to 9, wherein the deodorization device is installed in an installation hole (101) of the drum (100).
